Europäisches Patentamt

European Patent Office

Office européen des brevets

(19)

(11) Publication number: **0 314 016 B1**

# EUROPEAN PATENT SPECIFICATION

(12)

(45) Date of publication of patent specification: **23.12.92**  (51) Int. Cl.⁵: **A61J 1/00**

(21) Application number: **88117607.7**

(22) Date of filing: **21.10.88**

(54) **Switch bag type blood-gathering set.**

(30) Priority: **24.10.87 JP 267640/87**
**24.10.87 JP 267641/87**
**24.10.87 JP 267642/87**
**27.09.88 JP 241254/88**

(43) Date of publication of application:
**03.05.89 Bulletin 89/18**

(45) Publication of the grant of the patent:
**23.12.92 Bulletin 92/52**

(84) Designated Contracting States:
**DE FR GB IT**

(56) References cited:
**EP-A- 0 175 274       WO-A-87/06119**
**DE-A- 2 944 492       FR-A- 2 548 905**
**US-A- 3 460 789       US-A- 3 945 380**
**US-A- 4 014 329       US-A- 4 332 378**

(73) Proprietor: **KAWASUMI LABORATORIES, INC.**
**No. 28-15, Minami-Ohi, 3-chome Shinagawa-ku**
**Tokyo(JP)**

(72) Inventor: **Juji, Takeo,**
**No. 17-16,Tokiwadaira, 6-chome,**
**Matsudo-shi, Chiba-ken,(JP)**
Inventor: **Wakimoto, Nobuhiro,**
**No. 18-15-304, Horinouchi, 2-chome,**
**Suginami-ku, Tokyo,(JP)**
Inventor: **Kagawa, Yoichi, c/o Kawasumi Laboratories, Inc.,**
**No. 28-15, Minami-Ohi, 3-chome,**
**Shinagawa-ku, Tokyo,(JP)**
Inventor: **Ono, Seiichi, c/o Kawasumi Laboratories, Inc.,**
**Mie Kojo No. 7-1, Ohaza-Tamada, Mie-cho,**
**Ohno-gun, Ohita-ken,(JP)**

(74) Representative: **von Bezold, Dieter, Dr. et al**
**Dr. Dieter von Bezold Dipl.-Ing. Peter Schütz**
**Dipl.-Ing. Wolfgang Heusler Brienner Strasse 52**
**W-8000 München 2(DE)**

EP 0 314 016 B1

## Description

## BACKGROUND OF THE INVENTION

This invention relates to a switch bag type blood-gathering set to effect blood transfusion of stored blood and blood-gathering of fresh blood at the same time.

Blood gathered in a plastic bag should be obligated to use within 21 days, but, actually, it has been used within 7 days or so in many cases.

When the gathered blood is stored for a long time, hemolysis or formation of microaggregates in blood proceeds. It is not preferred to use such a blood for transfusion and the gathered blood should desirably be employed for transfusion as soon as possible.

Also, an amount of blood gathered from a blood donor at once is limited to in the range of 400 ml to 500 ml. This is because if a large amount of blood is gathered from a blood donor at once, effects to a living body, such as anemia, decrease in blood component, etc. are serious in view of balance of body fluids.

Further, fresh blood of other person includes a risk of infection of syphilis, hepatis, etc. so that it has been conducted to establish a method in which self-blood is transfused.

Particularly, to the patient who can be conducted an expected operation in addition to articulatio coxae shelf rotatory born cutting operation, scoliosis operation and artificial articulatio coxae whole replacement operation, and who can be capable of gathering blood, it is preferred to employ a technique for predeposit of autologus blood in which autologus blood is gathered and reserved, and the the stored blood is transfused at the operation.

For this method, the technique for predeposit of autologus blood gathered before an operation such as the refrigerator storing method or the Leapfrog method (liquid state storing method) has heretofore been employed.

For example, an example of the Leapfrog method will be explained by referring to Fig. 18.

As the first time, one unit of a blood A is gathered in a blood storing bag 500a (see Fig. 18(a)), and at the second time, as shown in Fig. 18(b),

(1) one unit of a blood B is gathered from a patient into a blood storing bag 500b,

(2) a blood A in the blood storing bag 500a is transfused to the patient, and

(3) one unit of a blood C is again gathered from the patient into a blood storing bag 500c,

whereby the blood B or the blood C in excess of one unit from that of the first time is gathered.

As the third time (see Fig. 18(c)),

(1) one unit of a blood D is gathered from the patient,

(2) one unit of the blood B gathered at the second time is transfused to the patient, and

(3) one unit of a blood E is gathered from the patient.

At the fourth time (see Fig. 18(d)),

(1) one unit of a blood F is gathered,

(2) one unit of the blood C gathered at the second time is transfused to the patient, and

(3) one unit of a blood G is gathered from the patient.

At the fifth time (see Fig. 18(e)),

(1) one unit of a blood H is gathered,

(2) one unit of the blood D gathered at the third time is transfused to the patient, and

(3) one unit of a blood I is gathered from the patient.

Thus, one unit of the blood gathered at the previous time is returned `and at the same time, two units of blood is gathered so that stored amounts of blood can be increased.

However, in the Leapfrog method, there are problems that (1) the stored amount of blood is limited, (2) oxygen transferability of the blood becomes low, and (3) blood-gathering term is limited, etc., and in the refregirator storing method, there are also problems in loss of blood cell at thawing, or in costs for storing and thawing.

From US-A-3 945 380 is known a plasmaferesis assembly for collecting blood from a donor, storing the collected blood in blood collection bags, sealing and separating the blood collection bags from the system, centrifuging the blood collection bags for separating blood plasma and blood cells, and re-introducing the blood cells to the donor, while the plasma is collected. For this purpose, the system of the citation includes blood-transfusing and blood-gathering means including a needle for gathering blood from and transfusing a solution of the blood cells back to the donor, and a plurality of blood-gathering bags for collecting the blood.

Further, FR-A-2 548 905 describes collecting blood from a donor, separating the collected blood into blood cells and plasma, and re-transfusing the blood cells back to the donor, similar to the system known from US-A-3 945 380.

**SUMMARY OF THE INVENTION**

The object of the present invention is to provide a blood-gathering set which can transfuse blood without loosing freshness of the blood gathered from the donor as well which can gather the blood in an larger amount with preserving balance of body fluid.

According to the present invention there is provided a blood gathering set of a switch-bag type for performing blood transfusion of blood, gathered from a blood donor and stored beforehand, back to the same blood donor, and storing fresh blood by performing blood-gathering from the blood donor again, said blood-gathering set comprising the features defined in claim 1.

**BRIEF DESCRIPTION OF THE DRAWINGS**

Fig. 1 is a schematic view showing a basic constitution of the switch bag type blood-gathering set of the present invention;

Fig. 2, Fig. 3 and Fig. 4 are schematic views showing modified examples of the switch bag type blood-gathering set shown in Fig. 1;

Fig. 5 (a) and (b) are schematic views showing other examples of a liquid transferring member and a blood transfusing member constituting a switch bag type blood-gathering set;

Fig. 6 is an example of a check valve provided to a washing solution storing member wherein (a) is a plane view and (b) is a sectional view of A-A;

Fig. 7 is anther example of the check valve as the same above wherein (a) is a plane view and (b) is a sectional view of B-B;

Fig. 8 is an enlarged view at the neighbour of a connecting tube of a washing solution storing member;

Fig. 9 is a schematic view for explaining a using example of male lure connector of a washing solution storing member;

Fig. 10 is a schematic view showing other example of a switch bag type blood-gathering set of the present invention;

Fig. 11 is a schematic view of a blood storing member in which the switch bag type blood-gathering set of Fig. 10 is used by connecting thereto;

Fig. 12 is a schematic view showing a modified example of the blood-gathering set of Fig. 10;

Fig. 13 is a schematic view showing other example of a connecting portion of a connecting member and a blood storing member of the switch bag type blood-gathering set of Fig. 10;

Fig. 14 is a schematic view of a switch bag type blood-gathering set to be used for a technique for predepodit of autologus blood storing method of the present invention;

Fig. 15 Fig. 16 and Fig. 17 are schematic views for explaining a technique for predeposit of autologus blood storing method of the present invention; and

Fig. 18(a) to (e) are schematic views showing the conventional technique for predeposit of autologus blood.

**DESCRIPTION OF THE PREFERRED EMBODIMENT**

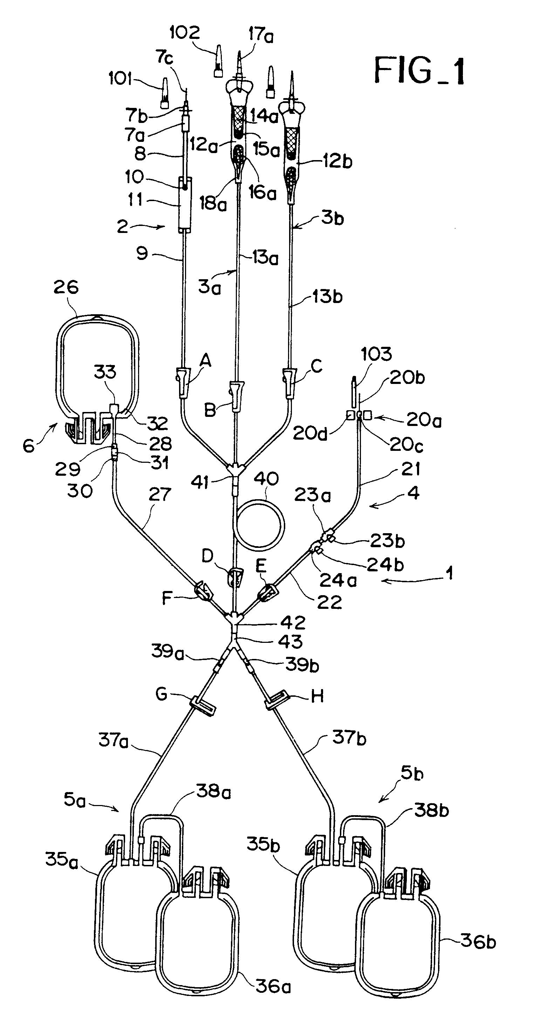

Fig. 1 is a schematic view showing one example of a switch bag type blood-gathering set of the present invention.

The switch bag type blood-gathering set 1 is basically constituted by a liquid transfer member 2, blood transfer members 3a and 3b, a blood-transfusing and blood-gathering member 4, blood storing members 5a and 5b, and a washing solution storing member 6.

The liquid transfer member 2 comprises a liquid introducing needle 7a which is provided a needle 7c made of a stainless steel, etc. to a needle base 7b, connecting tubes 8 and 9 made of a flexible polyvinyl chloride, etc., and an intravenous drip tube 11 made of a polyvinyl chloride, etc. loaded a filter 10 made of a polyamide such as Nylon (trade name), etc.

The blood transfer member 3a is composed of an intravenous drip tube 12a made of a polyvinyl chloride, etc. and a connecting tube 13a made of a flexible polyvinyl chloride, and said intravenous drip tube 12a is loaded inside thereof filters 14a, 15a and 16a made of a polyethyleneterephthalate, etc. having different pore size, respectively. Also, at the ends of the intravenous drip tube 12a, the blood introducing needle 17a made of a polycarbonate, etc. and the connecting tube 18a (made of polyvinyl chloride, etc.) with the above connecting tube 13a.

3

The above blood-transfusing and blood-gathering member 4 comprises a blood-transfusing and blood-gathering needle 20a in which a needle 20b made of a stainless steel, etc. is provided into a needle base 20c and a rotary wing 20d is further provided to the needle base 20c, and connecting tubes 21 and 22 made of a flexible polyvinyl chloride, etc. Between these two connecting tubes 21 and 22, mixing portions 23a and 24a made of a polycarbonate, etc. buried puncture buttons 23b and 24b made of a silicone rubber, etc. are attached.

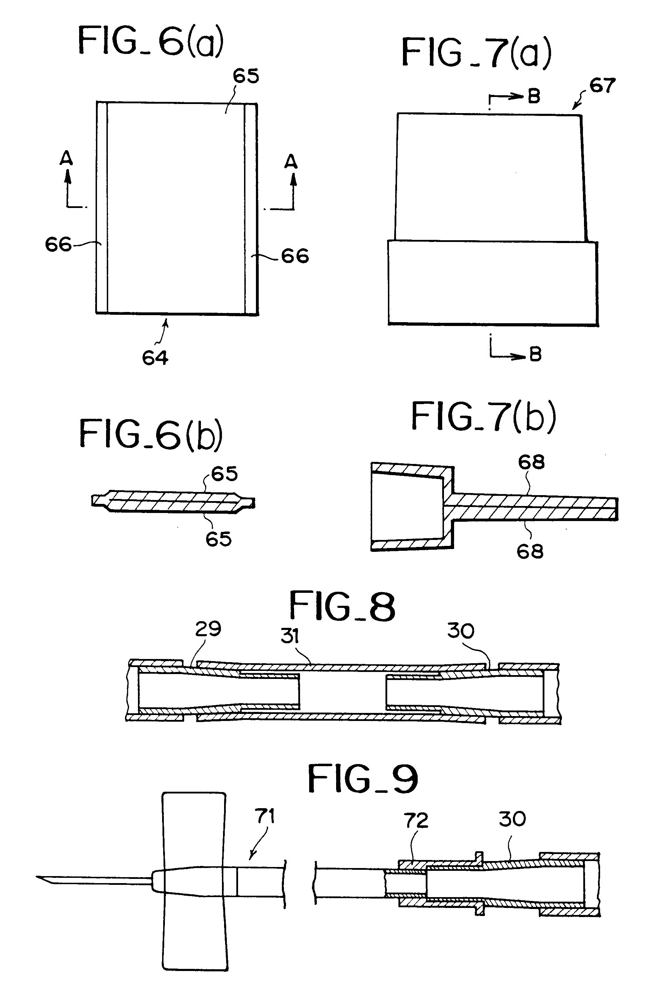

The washing solution storing member 6 is composed of a liquid discharging bag 26 made of a flexible polyvinyl chloride, etc. and connecting tubes 27 and 28 similarly made of a flexible polyvinyl chloride, etc. Each ends of said connecting tubes 27 and 28, male lure connectors 29 and 30 are provided as shown in Fig. 8, and both of the male lure connectors 29 and 30 are connected by a connecting tube 31 made of a silicone rubber, etc.

At a discharged liquid inlet 32 of the liquid discharging bag 26, a check valve 33 made of a flexible polyvinyl chloride or a silicone rubber, etc. is provided thereto.

The blood storing members 5a and 5b are composed of parent or main bags 35a and 35b, children or auxiliary bags 36a and 36b, and connecting tubes 37a, 37b, 38a and 38b, and connecting pieces 39a and 39b made of a polycarbonate, etc. are provided at inner portions of the above connecting tubes 37a and 37b. In these connecting pieces, a cylindrical body made of a hard resin and one end of which is terminated is encapsulated, and flow passages are sealed by the cylindrical bodies. When using it, one end terminated is broken whereby the flow passage is opened. Such connecting pieces have widely been utilized for blood transfusing site, etc. of the conventional blood-gathering bag.

In the above parent bags 35a and 35b, as an anticoagulant of the blood, solutions of

(1) CPD solution comprising citrate, phosphate and dextrose, and

(2) ACD solution comprising adenine, citrate and dextrose are charged.

These constituting members 2, 3a, 3b, 4, 5a, 5b and 6 are connected by connecting tubes 41, 42 and 43 made of a polyvinyl chloride through connecting tubes 9, 13a, 13b, 21, 22, 27, 28, 37a, 37b and 40, respectively.

At the connecting tubes 9, 13a and 13b, roll clamps A, B and C made of a polyethylene, etc. are provided.

At the connecting tubes 22, 27 and 40, clamps D, E and F made of a polypropylene, etc. are provided.

At the connecting tubes 37a and 37b, slide clamps G and H made of a polypropylene, etc. are provided.

The liquid introducing needle 7a, the blood introducing needle 17a and the blood-transfusing and blood-gathering needle 20a are sealed with needle caps 101, 102 and 103 made of a polypropylene, etc., respectively, so as to not contact with the air outside.

Next, the using method of the present invention will be explained by referring to Fig. 1.

(1) Priming operation

After closing the roll clamps A, B and C, and the clamps D, E and F, the liquid introducing needle 7a is connected to an apparatus (not shown in the figure) containing a physiological saline solution.

By opening the roll clamp A, the physiological saline solution is injected into an intravenous drip cylinder 11 to a predetermined liquid level with pumping the intravenous drip cylinder 11.

When reached to the predetermined liquid level, the roll clamp B is opened and the physiological saline solution is introduced into inside of an intravenous drip cylinder 12a throught the connecting tubes 9 and 13a. When reached to the predetermined liquid level, the roll clamp C is further opened and the physiological saline solution is also similarly introduced into the intravenous drip cylinder 12b with a predetermined liquid level.

Subsequently, clamps D, E and F are opened, physiological saline solutions are added dropwise while adjusting the opening degree of the roll clamp A in order to expel the air in the connecting tubes 40, 27 and 28 with the physiological saline solution into a discharged liquid bag 26 whereby priming is finished by closing the clamps D, E and F.

(2) Blood-gathering at the first time

By breaking the connecting piece 39a of the connecting tube 37a to open the flow passage, and after opening the slide clamp G, the blood-transfusing and blood-gathering needle 20a is punctured to a blood donor and the clamp E is opened.

The blood is flown into a parent bag 35a through the blood-transfusing and blood-gathering needle 20a,

4

and the connecting tubes 21, 22 and 37a.

After finishing collection of the predetermined amount of blood, closing the slide clamp G and opening the roll clamp A and the clamp D, the physiological saline solution is added dropwise from the liquid transferring member 2 into the blood-transfusing and blood-gathering member 4 through the connecting tube 40.

At this time, the blood-transfusing and blood-gathering needle 20a should be remained at the state of puncturing into the blood donor. This is to maintain the closed system after finishing the blood-gathering.

To do so, solidification due to accumulation of blood in the liquid transferring and blood-gathering member 4 during the transitional period to the next operation can be prevented.

(3) Blood-transfusing operation

A blood bag (not shown in the figure) in which blood is previously stored and contained therein by the prior blood-gathering is connected to the blood introducing needle 17a of the blood transfusing member 13a.

When the roll clamp B is opened and the roll clamp C is closed, the blood is transfused to a blood donor through the connecting tubes 13a, 40, 22 and 21 and the blood-transfusing and blood-gathering needle 20a. The blood transfusing rate at this time is preferably about 60 ml/min (the degree that an appearance running down to the center of instillation does not become a continuous line).

After finishing the transfusion of the blood in this blood bag, the roll clamp B is closed.

Then, the roll clamp A is opened again, the physiological saline solution is introduced into the blood-transfusing and blood-gathering member 4 in order to prevent solidification of the blood remained in the blood-transfusing and blood-gathering member 4.

(4) Blood-gathering at the second time.

By breaking the connecting piece 39b, the clamp H and the clamp E are opened.

The blood is introduced into the parent bag 35b through the blood-transfusing and blood-gathering needle 20a and the connecting tubes 21, 22 and 37b.

After gathering the predetermined amount of blood, closing the clamp E and opening the clamp A, the physiological saline solution is added dropwise from the liquid transferring member 2 to the blood-transfusing and gathering member 4.

(5) Termination

By closing the clamp E, an electrolyte previously prepared is supplemented to the blood donor through the mixing part 23a, the connecting tube 21 and the blood-transfusing and gathering needle 20a.

Finally, the connecting tubes 37a and 37b are sealed and welded by a welder, and then the blood storing members 5a and 5b are cut off to use for the next blood transfusion by storing them at 4 ° C.

As the other examples of the switch bag type blood-gathering set of the present invention, numbers of the blood-transfusing member(s) and the blood storing member(s) may optionally be combined.

For example, embodiments of

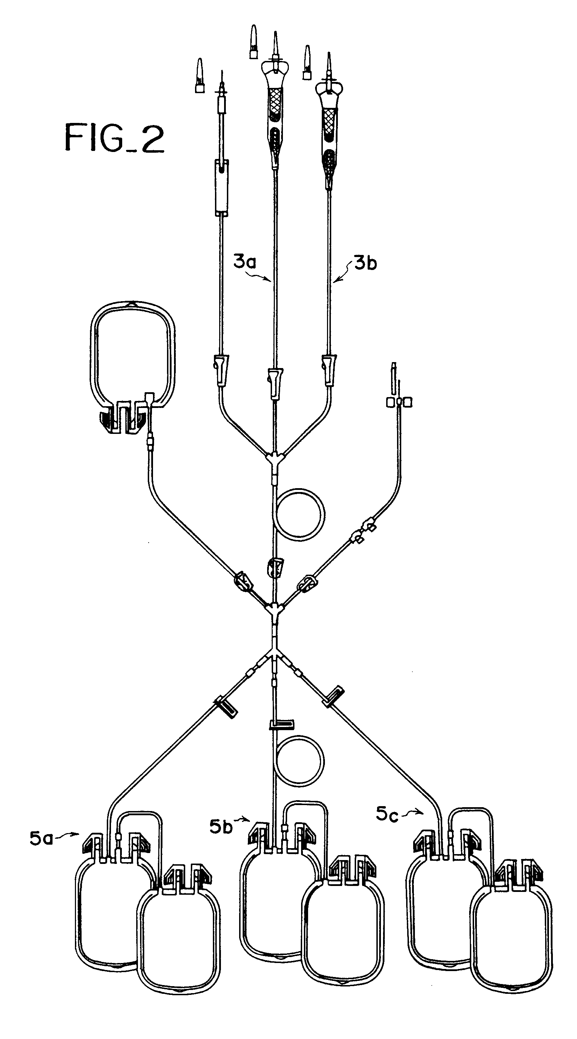

(1) two blood-transfusing members (3a and 3b) and three blood-storing members (5a, 5b and 5c) (see Fig. 2),

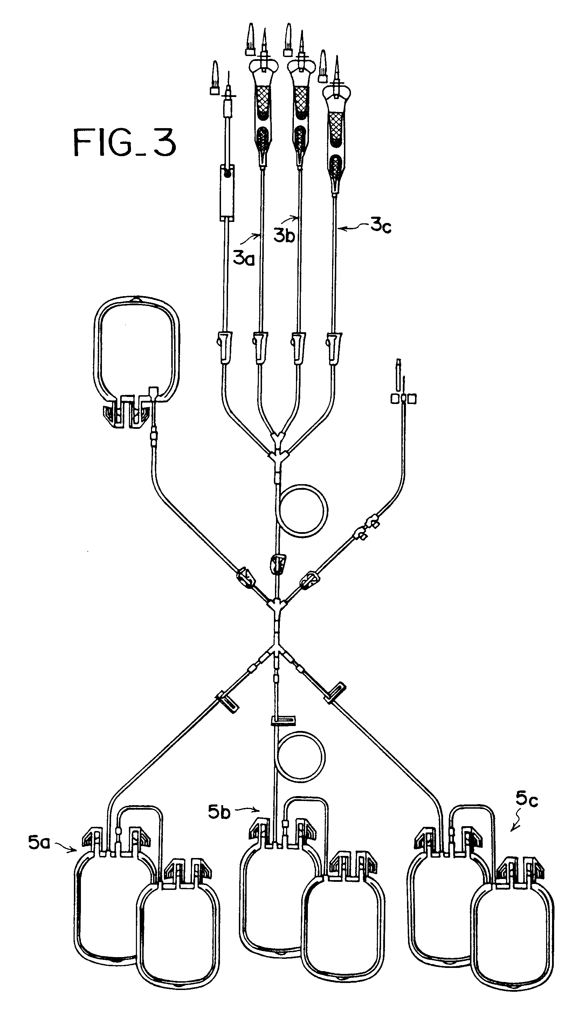

(2) three blood-transfusing member (3a, 3b and 3c) and three blood-storing members (5a, 5b and 5c) (see Fig. 3), and

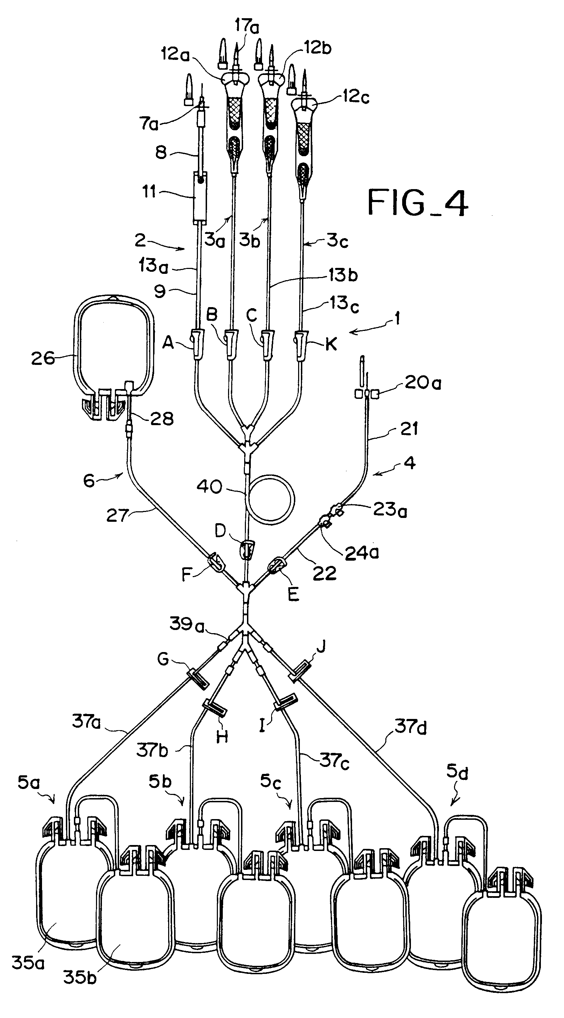

(3) three blood-transfusing member (3a, 3b and 3c) and four blood-storing members (5a, 5b, 5c and 5d) (see Fig. 4) may be considered.

Further, by combining numbers of the blood storing member (5a to 5d) and volumes of the parent bags (38a to 38d) in said blood storing member (5a to 5d), ten kinds of embodiments can be carried out.

As shown in Table 1, an amount of blood to be reserved can be determined before blood-gathering by combining blood-gathering patterns in accordance with the condition of health and physical strength of the blood donor.

Table 1

|  | Initial | Second time | Third time | Fourth time | Fifth time | Blood amount reserved |
|---|---|---|---|---|---|---|
| Example 1 | 200 ml x 1 | 200 ml x 2 | 300 ml x 2 | 400 ml x 2 | 500ml x 2 | 1000 ml |
| Example 2 | 300 ml x 1 | 300 ml x 2 | 300 ml x 3 | 400 ml x 3 | 500ml x 3 | 1500 ml |
| Example 3 | 400 ml x 1 | 400 ml x 2 | 400 ml x 3 | 400 ml x 4 | 500ml x 4 | 2000 ml |
| Example 4 | 200 ml x 2 | 200 ml x 2 | 200 ml x 2 |  |  | 800 ml |
| Example 5 | 200 ml x 2 | 200 ml x 2 | 200 ml x 3 |  |  | 1200 ml |

Remarks (1) Example 1 to Example 3 are examples of the method in which the blood gathered at the initial time is transfused at the next time and further blood is gathered excessively 200 ml to 400 ml, and these procesures are repeated.

(2) Example 4 and Example 5 are examples of the method in which blood-gathering of 200 ml X 2 is carried out at the initial time, and at the second time, the third time, etc., 200 to 400 ml of excessive amount of blood is gathered and these procedures are repeated.

(3) As the other methods, according to the system of switch bagging every two weeks by dividing examples of Example 1 to Example 3 into two system, a large amount of blood may be reserved. Also, in accordance with an amount of hemoglobin before blood-gathering of the blood donor may be reserved.

(4) While Example 1 to Example 5 are basic examples, by using this system, larger amount of blood can be reserved by increasing the number of times or combining Example 1 to Example 5.

Next, modified examples of each part of the switch bag type blood-gathering set of the present invention will be explained.

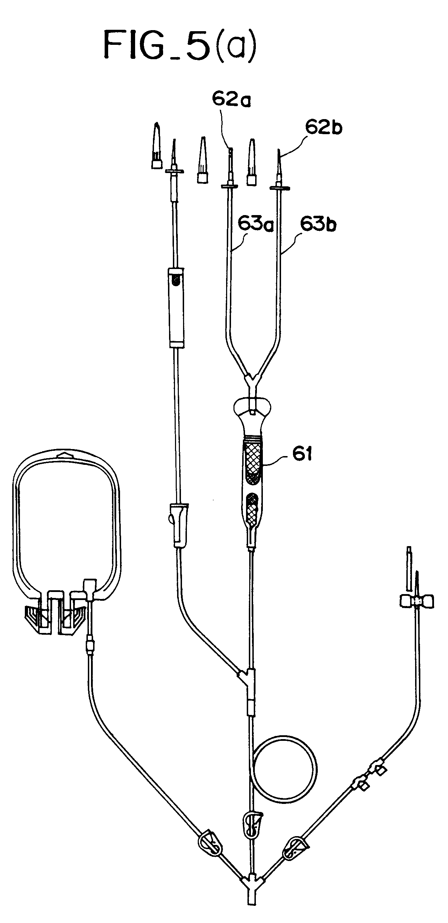

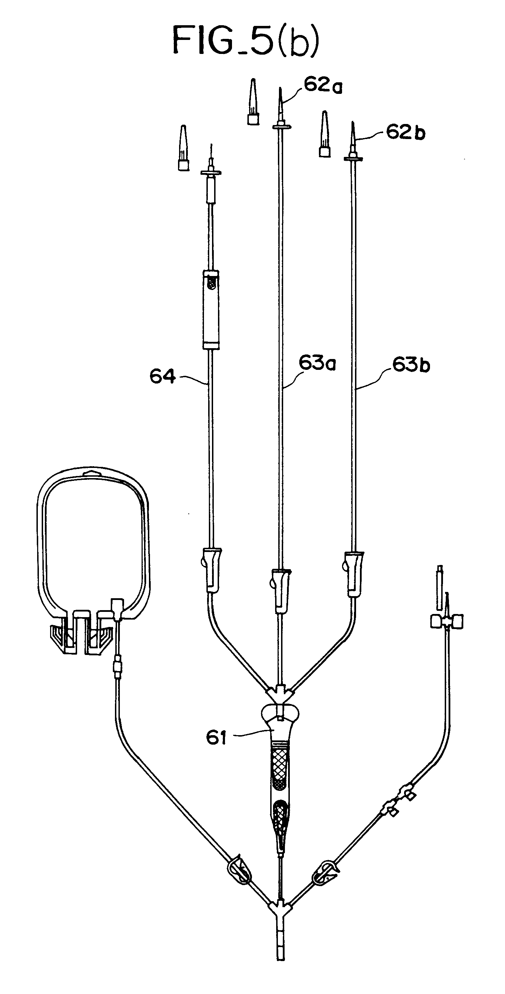

When the blood transfusing member is two pair or more, as shown in Fig. 5(a), plural numbers of the connecting tubes 63a and 63b in which the blood inlet needles 62a and 62b are provided may be fit to one intravenous drip cylinder 61. Or else, as shown in Fig. 5(b), the connecting tube 64 of the liquid transferring member may be provided to the intravenous drip cylinder with the aforesaid connecting tubes 63a and 63b.

By this constitution, numbers of parts just omitted intravenous drip cylinders can be reduced.

Concrete embodiments of the check valve 33 formed at the washing solution storing member 6 are shown in Fig. 6 and Fig. 7.

A check valve 64 of Fig. 6 is formed by laminating two sheets 65 made of a flexible polyvinyl chloride and heat sealing ends 66 thereof.

A check valve 67 of Fig. 7 is a generally so-called "duck's bill valve" and is integrally formed by a flexible polyvinyl chloride or a silicone rubber. Reference numeral 68 is a bill portion formed to a thin state.

By providing these check valves 64 and 67 to a waste solution inlet 32 of a wast liquid bag 26, when pressure is applied from outside of the waste liquid bag 26 after introducing a washing solution in the waste liquor bag 26, said external pressure functions to the direction of crushing the sheets 65 and 65 of the check valve 64 or the bill portion 68 of the check valve 67, whereby sanitariness can be retained without invading again the used washing solution into inside of the switch bag type blood-gathering set.

The male lure connectors 29 and 30 provided at the ends of the connecting tubes 27 and 28 of the washing solution storing member 6 can be used as shown below.

When the blood donor is punctured with the blood-transfusing and gathering needle 20a to carry out blood-gathering, if the trouble due to clogging of blood cells at inside of the needle 20b of the blood-transfusing and gathering needle 20a is caused, the connecting tube 31 is detached and the male lure connector 30 is exposed. Next, this male lure connector 30 is connected, for example, as shown in Fig. 9, to a female lure connector 72 of the blood-gathering needle 71 which is prepared for a spare and the blood donor is punctured with the blood-gathering needle 71.

The blood is introduced into the parent bag 35a of the blood storing member 5a through the connecting tubes 27 and 37a. To the contrary, the blood-transfusing and gathering needle 20a is employed for disposing waste luquor by connecting with the other male lure connector 29.

Pore sizes of filters 14a, 15a and 16a charged at inside of the intravenous drip cylinder 12a are formed as 210 $\mu$m, 160 $\mu$m and 35 $\mu$m, respectively, so as to become smaller from the inlet of the blood to the outlet direction. By this constitution, it can be completely prevented to invade microaggregates in the blood into the switch bag type blood-gathering set.

As the method for attaching the filters 14a, 15a and 16a to inside of the intravenous drip cylinder 12, the following methods may be mentioned.

(1) 14a and 15a are laminated and welded in the intravenous drip cylinder, and 16a is welded and fit to the bottom end of the intravenous drip cylinder.

(2) 14a, 15a and 16a are laminated together, and welded and fit to inner surface fo the intravenous drip cylinder.

A children bag 36a in the blood storing member 5a (which is fit to the parent bag 35a through the connecting tube 38a) is employed when the blood gathered in the parent bag 35a is sampled to apply to the blood inspection, etc. It is particularly useful for the blood inspection which requires a large amount of a sample.

As stated above, since the blood-gathering is effected by repeating blood-gathering and blood-transfusion wherein the amount of gathered blood is relatively somewhat larger than the transfused blood and supplementation of the blood corresponding to the amount of the blood gathered from the blood donor is carried out to the blood donor, the burden imposed on the blood donor is little.

Also, the numbers of a liquid introducing needle 7a, a blood introducing needle 17a and the blood-transfusing and gathering needle 20a or the shape of the connecting tubes 4, 42 and 43, and the materials of each constituting members 2, 3a, 4, 5a and 6 can optionally be determined depending upon purposes.

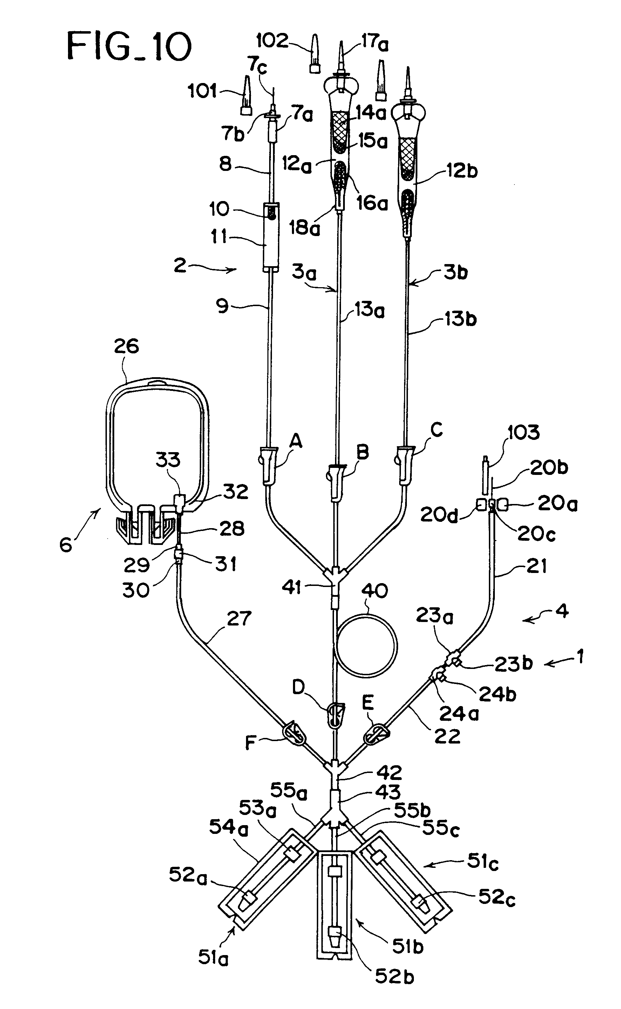

Fig. 10 is an other example of the present invention, and the difference from that of Fig. 1 is to connect connecting members 51a, 51b and 51c with the connecting tube 43 in place of connecting with the blood storing members 5a and 5b.

The connecting member 51a (51b and 51c are also the same) is composed of a connector member, i.e. a male connector 52a, and a connecting tube 55a, a lock nut 53a of said male connector 52a is freely fit. The male connector 52a and the lock nut 53a are sealed by a protector 54a so as to not contacting with the air outside.

The connecting member 51a is constituted by a material such as a polyvinyl chloride, a polypropylene, a polycarbonate, etc.

These connecting members are connected with the connecting tube 42 through the connecting tube 43 made of a hard polyvinyl chloride, etc.

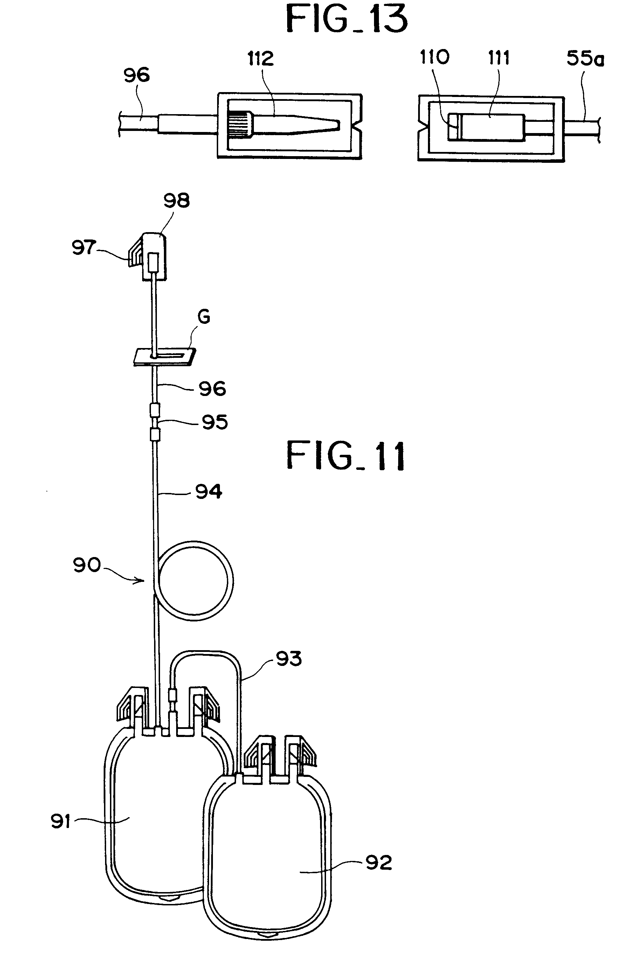

Fig. 11 is the aforesaid blood storing member 90 to be connected with the aforesaid connecting members 51a, 51b and 51c. Reference numeral 91 is a parent bag made of a flexible polyvinyl chloride, etc., and similarly, 92 is a children bag, and they are connected with each other through a connecting tube 93.

In the above parent bag 91, the aforesaid blood anticoagulant containing the above components are charged.

At the top end of the above parent bag 91, connecting tubes 94 and 96 are joined and at the top end of the connecting tube 96, a female connector is provided. This female connector is constituted by a material such as a polyvinyl chloride, a polypropylene, a polycarbonate, etc. Also, at the midportion of the above connecting tubes 94 and 96, a connecting pieces 95 made of a polycarbonate is fit, and to the connecting tube 96, the slide clamp G made of a polypropylene, etc. is fit.

A female connector 97 is sealed by a protector 98 so as to not contacting with the air outside.

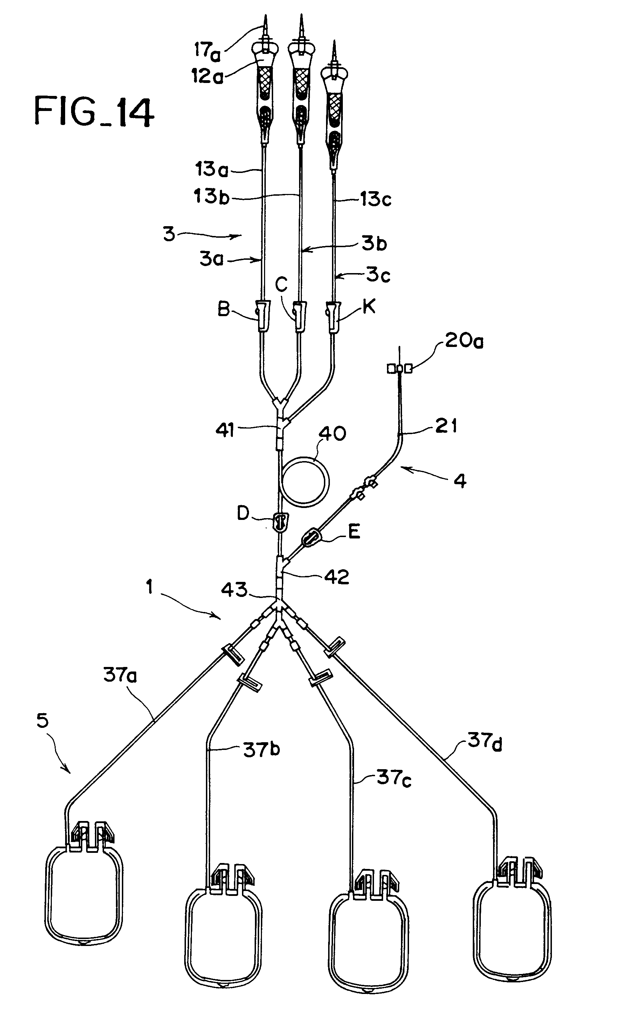

As the other examples of the above connecting memebers 5a, 5b and 5c, for example, as shown in Fig. 13, a female connector 111 (which is used as a blood transfusing inlet of the usual blood bag) inside of which is formed a thin film 110 is connected to a connecting tube 55a, and to a connecting tube 96 of the blood storing member 90, a needle member 112 may be provided. The needle member 112 is a plastic spike used in the conventional blood transfusing or liquid transferring set and it can break a thin film 110 when inserting it into the above female connector 111. The needle member 112 may be sealed with a needle cap in addition to a protector.

Next, using method of examples in Fig. 10 and Fig. 11 will be explained.

(1) Priming operation

The same as the aforesaid priming operation in Fig. 1.

(2) Blood-gathering at the first time

By breaking protectors 54a and 98, a male connector 52a of a connecting member 51a and a female connecctor 97 of a blood storing member 90 are connected with each other. At this time, in order to connect both connectors more fixedly, the connecting portion is tightened with a rock nut 53a.

By opening a connection piece 95 provided at the midportion of a connecting tube 94 to open blood flow passage of the connecting tubes 55a and 94, and puncturing the blood donor with a blood-transfusing and gathering needle 20a, a clamp E and a clamp G are opened.

By this constitution, the blood is introduced into a parent bag 91 through connecting tubes 21, 22, 55a, 96 and 94.

After completion of a predetermined amount of blood-gathering, the slide clamp G which is provided at the midportion of the connecting tube 96 is closed.

Thereafter, in the same manner as in the example of Fig. 1, a physiological saline solution is added dropwise into the blood-transfusing and gathering member 4.

(3) Blood transfusing operation

The same as in the operation of Fig. 1.

(4) Blood-gathering at the second time

New blood storing member 99 is prepared (which is other blood storing member than the blood storing member used in the blood-gathering at the first time).

Similarly as in the blood-gathering at the first time, blood is introducced from a blood donor into a new blood storing member.

After gathering a predetermined amount of blood, the clamp E is closed and a clamp A is opened, and a physiological saline solution is added dropwise from a liquid transferring member 2 into a blood-transfusing and gathering member 4.

(5) Termination

Similarly as in the operation in the example of Fig. 1, an electrolyte is supplemented to the blood donor.

Finally, the connecting tube 96 is sealed and welded by a welder, and then the blood storing member 90 is cut off to use for the next blood transfution by storing it at 4 °C.

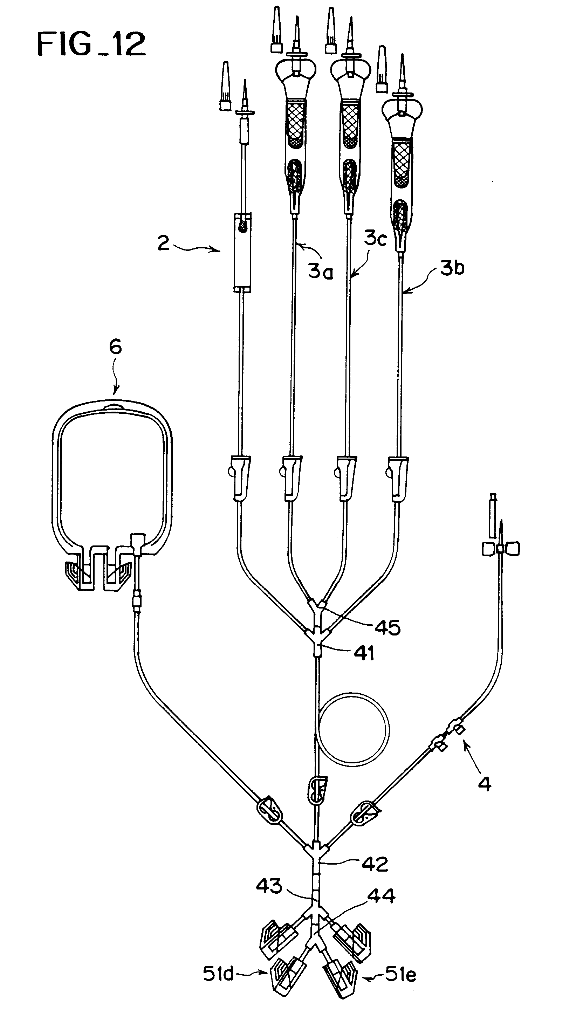

As the modified example of Fig. 10, as shown in Fig. 12, by newly adding connecting tubes 44 and 45, a blood transfusing member 3c and connecting members 51d and 51e can be increased. As the protector, other than the embodiment shown in Fig. 1, the protector used in a blood transfusion inlet of the conventional blood bag may be used.

As the switch bag type blood-gathering set to be used for a technique for predeposit of autologus blood, in addition to the aforesaid examples, a switch bag type blood-gathering set as shown in Fig. 14 can be used.

This blood-gathering set is a modified example of the switch bag type blood-gathering set shown in the above Fig. 4. Difference from Fig.4 is to omit the liquid transferring member 2, the washing solution storing member and each children bags of the blood storing members 5a, 5b, 5c and 5d. Other constitution is the same with that of Fig. 4.

Capacity and number of the blood transfusing member 3 and the blood storing member 5 may optionally be selected.

Next, an application example of the switch bag type blood-gathering set shown in Fig. 14 will be explained in the following.

First application example

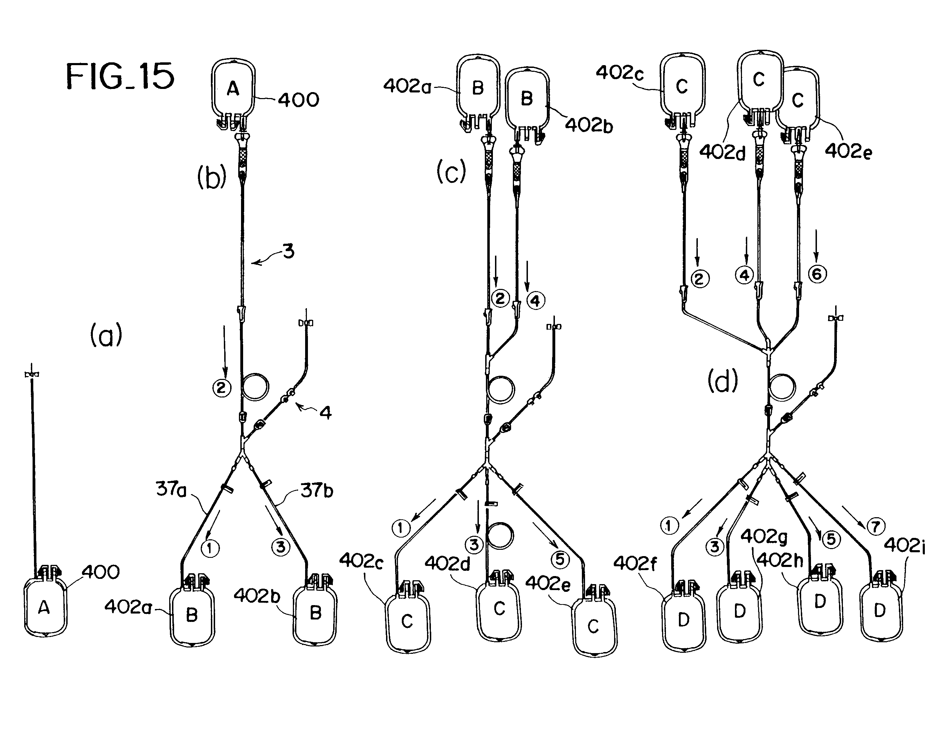

At the first time, one unit of a blood A is gathered in a blood storing bag 400 (see Fig. 15(a)).

At the second time, as shown with an arrow in Fig. 15(b),

(1) one unit of a blood B is gathered through a blood-transfusing and gathering member 4 via a connecting tube 37a into a blood storing bag 2a,

(2) the blood A gathered at the first time is transfused to the patient through a blood transfusing member 3 and the blood-transfusing and gathering member 4, and

(3) in the same manner as in (1), a blood B is gathered from the patient through the blood-transfusing and gathering member 4 and an inlet tube 37b into a blood storing bag 402b,

whereby the blood B in excess of one unit than that of the first time is gathered.

8

At the third time as in the second time as shown with an arrow in Fig. 15(c),

(1) one unit of a blood C is gathered from the patient into a blood storing bag 402c,

(2) one unit of the blood B is transfused to the patient from the blood storing bag 402a,

(3) one unit of a blood C is gathered from the patient into a blood storing bag 402a,

(4) one unit of the blood B is transfused to the patient from the blood storing bag 402b, and

(5) one unit of a blood C is gathered from the patient into a blood storing bag 402e.

At the fourth time as in the above as shown with an arrow in Fig. 15(d),

(1) one unit of a blood D is gathered from the patient into a blood storing bag 402f,

(2) one unit of the blood C is transfused to the patient from the blood storing bag 402c,

(3) one unit of a blood D is gathered from the patient into a blood storing bag 402g,

(4) one unit of the blood C is transfused to the patient from the blood storing bag 402d,

(5) one unit of a blood D is gathered from the patient into a blood storing bag 402h,

(6) one unit of the blood C is transfused to the patient from the blood storing bag 402e, and

(7) one unit of a blood D is gathered from the patient into a blood storing bag 402i.

However, each blood-gathering should be carried out with the interval of within the effective preservation term of the blood (within 21 days from blood-gathering).

As described above, the present application example is the technique for predeposit of autologus blood in which n units of bloods gathered from the first time to the Nth time are transfused to the patient at the (N + 1)th time and at the same time, (n + 1) units of blood are gathered.

By the operation as mentioned above, the patient is always gathered only in excess of one unit of blood, and the blood gathered at the Nth time is certainly transfused to the patient at the (N + 1)th time so that a large amount of fresh blood can be always deposited.

Second application example

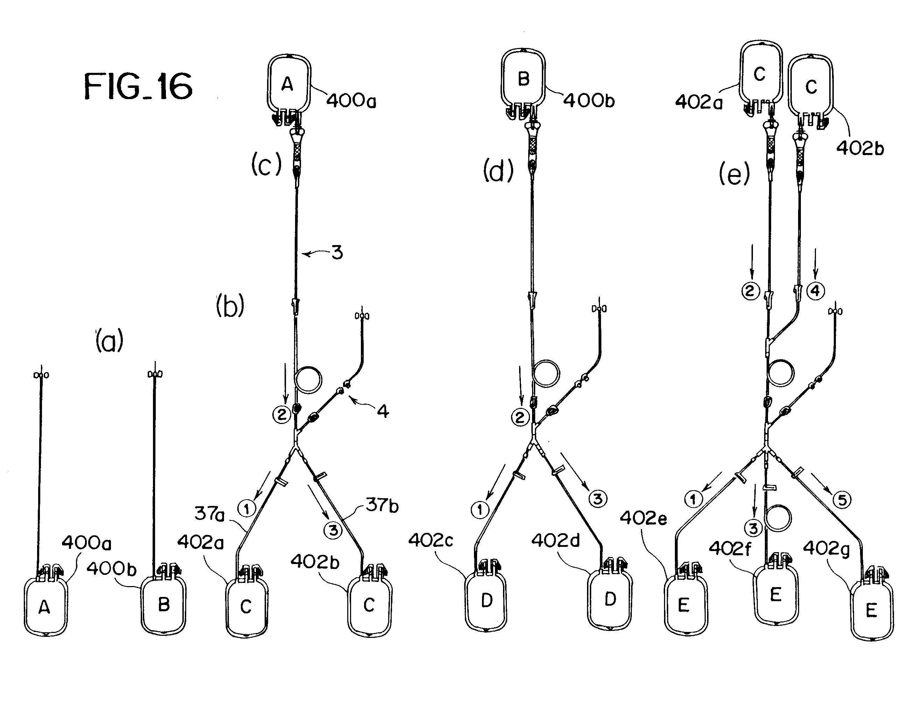

In the same manner as in the first application example, each one unit of bloods A and B is gathered in blood storing bags 400a and 400b at the first and the second times, respectively (see Fig. 16(a) and (b)).

At the third time, as shown with an arrow in Fig. 16(c),

(1) one unit of a blood C is gathered from a patient through a blood-transfusing and gathering member 4 via an inlet tube 37a into a blood storing bag 402a,

(2) one unit of the blood A gathered at the first time is transfused to the patient through a blood transfusing member 3 and the blood-transfusing and gathering member 4, and

(3) a blood C is gathered from the patient into a blood storing bag 402b,

At the fourth time as shown with an arrow in Fig. 16(d),

(1) one unit of a blood D is gathered from the patient into a blood storing bag 402c,

(2) one unit of the blood B gathered at the second time is transfused to the patient, and

(3) one unit of a blood D is gathered from the patient into a blood storing bag 402d.

At the fifth time as shown with an arrow in Fig. 16(e), three units of blood E are gathered from the patient as well as two units of blood C gathered at the third step are transfused, and the procedures are carried out alternately.

As described above, the present application example is a technique for predeposit of autologus blood in which n units of blood gathered at the Nth time are transfused to the patient at the (N + 2)th time as well as (n + 1) units of blood are gathered from the patient and stored, and said blood are transfused to the patient at the [(N + 2) + 2]th time.

Thus, in the second application example, since the first application example is divided into two series and blood-gathering is carried out, as compared with the first application example, number of the blood storing bag(s) used at the time carrying each operation does not change so that the time spent to work can be shortened and a large amount of predeposit of blood can be realized.

However, the blood-gathering time of each one series such as the first time and the third time, etc., it should be carried out within interval during effective preservation term of blood (within 21 days from blood-gathering).

Third application example

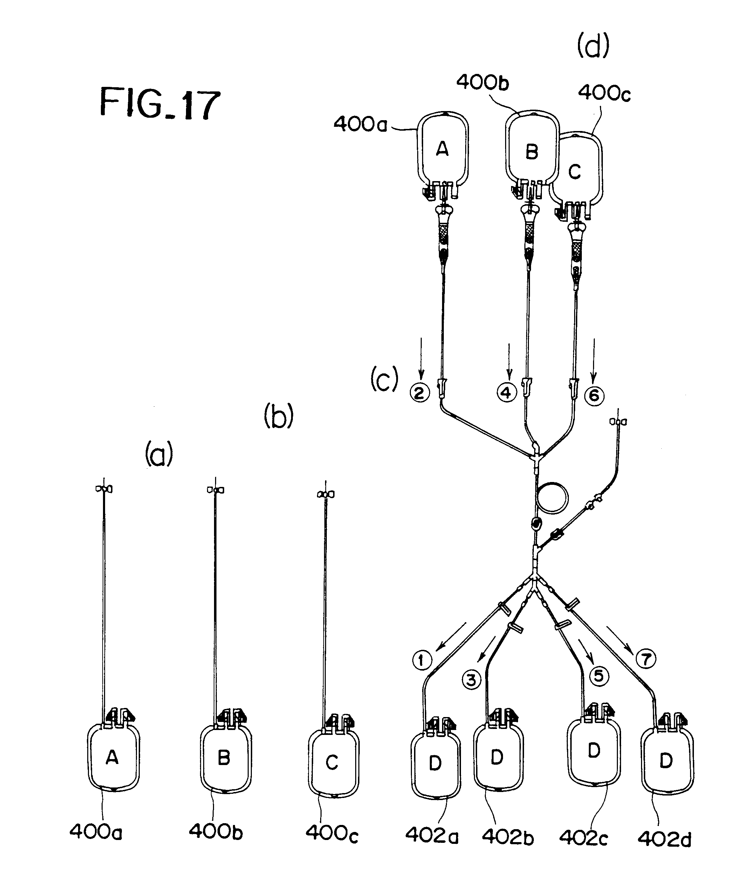

As shown in Fig. 17(a) to (c), each one unit of blood A, B and C (total three units of blood ) are gathered in blood storing bags 400a, 400b and 400c, respectively, at the first to the third times, and at the fourth time, as shown with an arrow in Fig. 17(d),

(1) one unit of a blood D is gathered from the patient into a blood storing bag 402a,

(2) one unit of the blood A gathered at the first time is transfused to the patient,

(3) one unit of a blood D is gathered from the patient into a blood storing bag 402b,

(4) one unit of the blood B gathered at the second time is transfused to the patient,

(5) one unit of a blood D is gathered from the patient into a blood storing bag 402c,

(6) one unit of the blood C gathered at the third time is transfused to the patient, and

(7) one unit of a blood D is gathered from the patient into a blood storing bag 402d.

As described above, the present application example is a technique for predeposit of autologus blood in which n units of blood gathered at from the first time to the Nth time are transfused to the patient at the (N + 1)th time as well as (n + 1) units of blood are gathered from the patient.

Thus, the whole amounts of blood gathered at the last time are returned to the patient and bloods in excees of one unit are newly gathered from the patent (intervals from the first blood-gathering to the final time should be within 21 days which are effective preservation term of bloods).

By using this method, fresh blood can relatively easily be stored within the limited range.

In the blood storing bags (400, 400a to 400c and 402a to 402i) shown in the above Fig. 15 to Fig. 17, an anticoagulant of blood having the composition as mentioned above is charged.

As described above, the present invention is an excellent invention having the effects of

(1) since blood gathered from the blood donor can be transfused without losing freshness as well as larger amounts of blood than those transfused to the donor can be gathered from the donor without preventing balance of body fluid, and they can be used with a recycle system, it is the most suitable for autologus blood of the donor,

(2) since each of constituting members is integrally connected whereby operation can be carried out with a closed system and thus sanitation can be secured,

(3) a certain amount (100 to 500 ml) of blood can be gathered with one blood-gathering operation and hepatic function is normal with the degree of metabolizing citric acid in the stored blood, and also it is efffective for an operation of a patient who has not trouble drived from blood (such as orthopaedic operation), and

(4) it is also effective in the case where a patient has a rare blood type and it can be judged that a sufficient amount of blood cannot be secured for operation.

## Claims

1. A blood-gathering set of a switch-bag type for performing blood transfusion of blood, gathered from a blood donor and stored beforehand, back to the same blood donor, and storing fresh blood by performing blood-gathering from the blood donor again, said blood-gathering set comprising:

    a) blood-transfusing and blood-gathering means (4) including a gathering needle (20a), for performing both the transfusion and the gathering of blood to and from the same blood donor, a first connecting tube (21,22) connected to the gathering needle (20a) for guiding blood, and a medicament solution introducing portion (23a,24a) connected to the first connecting tube (21,22) at a midportion of the first connecting tube (21,22);

    b) n individual blood-storing members (5a,5b) (n ≥ 2) each including a blood-gathering bag (35a,35b) for storing blood, a second connecting tube (37a,37b) for communicating blood-transfer means (3a,3b) and blood-gathering means (4) with the blood-gathering bag (35a,35b), the second connecting tube having a connecting piece (39a,39b) with a flow channel which is open when the set is in use, and

    c) a plurality of joining members (42,43) associated with the liquid transfer from the blood-transfusing and blood-gathering means (4) to the blood-storing members (5a,5b) for preventing invasion of outside air therein;

        **characterized** in that it further comprises:

    d) liquid transfer means (2) including a liquid introducing needle (7a) connectable to a container in which a medicament solution such as physiological salt solution or the like is stored, a third connecting tube (8,9) connected to the liquid introducing needle (7a), and a drip cylinder (11) connected to the third connecting (8,9) tube at a midpoint of the third connecting tube (8,9);

    e) in said blood-transfer means (3a,3b), N individual (N ≥ 2 and N ≤ n) blood-introducing needles (17a) connectable to blood bags (35a,35b) in which blood from the blood donor is stored for introducing back to the donor, drip cylinder means (12a,12b) being connected with the blood introducing needles (17a) and provided with filter means (14a,15a,16a) inside thereof, and a fourth connecting tube (13a,13b) being connected with the drip cylinder means (12a,12b); and

    f) washing solution storing means (6) including a waste liquor bag for recovering waste liquor after

EP 0 314 016 B1

priming of the blood-gathering set, and a fifth connecting tube (27,28) for guiding-waste liquor to inside of the waste liquor bag (26);

said individual blood-storing members (5a,5b) including preferably in addition a second bag (36a,36b) connected with the blood-gathering bag (35a,35b).

2. A blood-gathering set according to claim 1, comprising a connector (41) connected with said third and fourth connecting tubes (8,9) (13a,13b), connecting means for communicating the connector (42,43) and the first, second, and fifth tubes (21,22) (37a,37b) (27,28) with each other, and a sixth tube (40) for connecting the connector (41) with the connecting means.

3. A blood-gathering set according to claim 2, wherein each of said first, second, fifth, and sixth connecting tubes is provided with clamping means (A-H).

4. A blood-gathering set according to claim 1, 2 or 3, wherein the washing solution storing means (6) includes an element (31) for connecting the waste liquor bag (26) with the fifth connecting tube (27,28).

5. A blood-gathering set according to one of claims 1 to 4, wherein the waste liquor bag (26) has a valve (33) in the inside thereof for preventing countercurrent.

6. A blood-gathering set according to one of claims 2 to 5, further comprising n connector elements (51a,51b,51c) for removably connecting the individual blood-storing members (5a,5b) with the connecting means.

7. A blood-gathering set according to one of claims 1 to 6, wherein the blood-transfusing means is provided with a cylindrical instillator (61) which is connected to N individual ($N \geq 2$) blood introducing needles (62a,62b) in branching, the relation between the number N of the blood introducing needles (62a,62b) and the number n of the blood storing members (5a,5b) being $n \geq N$.

**Patentansprüche**

1. Satz zum Sammeln von Blut vom Typ mit wechselbarem Beutel zur Durchführung einer Bluttransfusion von vorher von einem Spender gesammeltem und aufbewahrtem Blut zurück zu demselben Blutspender, und Aufbewahrung von frischem Blut durch wiederum Durchführung des Blutsammelns von dem Blutspender, wobei der Blutsammelsatz enthält:

a) eine Bluttransfusions- und Blutsammeleinrichtung (4) enthaltend eine Sammelnadel (20a) zur Durchführung sowohl der Transfusion und des Sammelns von Blut zu und von demselben Blutspender, einen zum Führen von Blut mit der Sammelnadel (20a) verbundenen ersten Verbindungsschlauch (21,22), sowie einen Medikamentenlösungseinführungsbereich (23a,24a), der mit dem ersten Verbindungsschlauch (21,22) in einem Mittelbereich des ersten Verbindungsschlauches (21,22) verbunden ist;

b) n einzelne Blutaufbewahrungselemente (5a,5b) ($n \geq 2$) jeweils enthaltend einen Blutsammelbeutel (35a,35b) zur Aufbewahrung von Blut, einen zweiten Verbindungsschlauch (37a,37b) zur Verbindung einer Blutübertragungseinrichtung (3a,3b) und Bluttransfusionseinrichtung (4) mit dem Blutsammelbeutel (35a,35b), wobei der zweite Verbindungsschlauch ein Verbindungsstück (39a,39b) mit einem bei der Benutzung des Satzes offenen Strömungskanal aufweist, und

c) eine Anzahl von der Flüssigkeitsübertragung von der Bluttransfusions- und Blutsammeleinrichtung (4) zu den Blutaufbewahrungselementen (5a,5b) zugeordneten Verbindungselementen (42,43) zur Verhinderung eines Eindringens von Außenluft dorthinein;

**dadurch gekennzeichnet,** daß er weiterhin enthält:

d) eine Flüssigkeitsübertragungseinrichtung (2) enthaltend eine mit einem Behälter, in welchem eine Medikamentenlösung wie eine physiologische Salzlösung oder dergleichen gespeichert ist, verbindbare Flüssigkeitseinführungsnadel (7a), einen mit der Flüssigkeitseinführungsnadel (7a) verbundenen dritten Verbindungsschlauch (8,9), sowie einen an einem Mittelbereich des dritten Verbindungsschlauches (8,9) mit dem dritten Verbindungsschlauch (8,9) verbundenen Tropfzylinder (11);

e) in der Blutübertragungseinrichtung (3a,3b) vorgesehene N einzelne ($N \geq 2$ und $N \leq n$) Bluteinführungsnadeln (17a), die mit Blutbeuteln (35a,35b), in welchen Blut von dem Blutspender für die Rückführung zu dem Spender gespeichert ist, verbindbar sind, mit den Bluteinführungsnadeln (17a) verbundene und mit Filtereinrichtungen (14a,15a,16a) in ihrem Inneren versehene Tropfzylinderein-

11

richtungen (12a,12b), sowie einen mit der Tropfzylindereinrichtung (12a,12b) verbundenen vierten Verbindungsschlauch (13a,13b); und

f) eine Waschlösungsaufbewahrungseinrichtung (6) enthaltend einen Abfallflüssigkeitsbeutel zum Zurückerlangen von Abfallflüssigkeit nach dem Vorspülen des Blutsammelsatzes, sowie einen fünften Verbindungsschlauch (27,28) zum Führen von Abfallflüssigkeit in das Innere des Abfallflüssigkeitsbeutels (26);

wobei die einzelnen Blutaufbewahrungselemente (5a,5b) vorzugsweise zusätzlich einen mit dem Blutsammelbeutel (35a,35b) verbundenen zweiten Beutel (36a,36b) enthalten.

2. Blutsammelsatz nach Anspruch 1, enthaltend ein mit den dritten und vierten Verbindungsschläuchen (8,9) (13a,13b) verbundenes Verbindungsstück (41), eine Verbindungseinrichtung für eine Verbindung des Verbindungsstücks (42,43) und der ersten, zweiten und fünften Schläuche (21,22) (37a,37b) (27,28) miteinander, sowie einen sechsten Schlauch (40) zur Verbindung des Verbindungsstücks (41) mit der Verbindungseinrichtung.

3. Blutsammelsatz nach Anspruch 2, bei dem die ersten, zweiten, fünften und sechsten Verbindungsschläuche mit Klemmeinrichtungen (A-H) versehen sind.

4. Blutsammelsatz nach Anspruch 1, 2 oder 3, bei dem die Waschlösungsaufbewahrungseinrichtung (6) ein Element (31) zur Verbindung des Abfallflüssigkeitsbeutels (26) mit dem fünften Verbindungsschlauch (27,28) enthält.

5. Blutsammelsatz nach einem der Ansprüche 1 bis 4, bei dem der Abfallflüssigkeitsbeutel (26) in seinem Inneren ein Ventil (33) zur Verhinderung einer Gegenströmung aufweist.

6. Blutsammelsatz nach einem der Ansprüche 2 bis 5, weiterhin enthaltend n Verbindungselemente (51a,51b,51c) für eine lösbare Verbindung der einzelnen Blutaufbewahrungselemente (5a,5b) mit der Verbindungseinrichtung.

7. Blutsammelsatz nach einem der Ansprüche 1 bis 6, bei dem die Bluttransfusionseinrichtung mit einer zylindrischen Einträufeleinrichtung (61) versehen ist, welche mit N einzelnen (N ≧ 2) Bluteinführungsnadeln (62a,62b) in Abzweigung verbunden ist, wobei die Beziehung zwischen der Anzahl N der Bluteinführungsnadeln (62a,62b) und der Anzahl n der Blutaufbewahrungselemente (5a,5b) n ≧ N ist.

## Revendications

1. Dispositif de prélèvement de sang du type à poche interchangeable pour la réalisation d'une transfusion de sang recueilli chez un donneur de sang et stockè préalablement, retour au même donneur de sang et stockage du sang frais en effectuant à nouveau une prélèvement de sang à partir du même donneur de sang, ce dispositif de prélèvement de sang comprenant:

a) des moyens de transfusion de sang et de prélèvement du sang (4) comprenant une aiguille de prélèvement (20a) pour réaliser à la fois la transfusion de sang vers et le prélèvement du sang du même donneur de sang, un premier tube de liaison (21, 22) relié à l'aiguille de prélèvement (20a), pour l'acheminement du sang, et une portion d'introduction d'une solution de médicament (23a, 24a) relié au premier tube de liaison vers le milieu du premier tube de liaison (21,22);

b) n éléments individuels de stockage de sang (5a, 5b) (n ≧ 2) comprenant chacun une poche de prélèvement (35a, 35b) pour stocker le sang, un second tube de liaison (37a, 37b) pour mettre en communication le moyen de transfert de sang (3a, 3b) et le moyen de prélèvement de sang (4) avec la poche de prélèvement de sang (35a, 35b) le second tube de liaison possédant une pièce de connexion (39a, 39b) comportant un canal d'écoulement qui est ouvert quand le dispositif est utilisé ; et

c) une pluralité d'éléments de connexion (42, 43) associés au transfert de liquide à partir des éléments de transfusion et de prélèvement du sang (4) vers les éléments de stockage du sang (5a, 5b) pour éviter l'entrée d'air extérieur;

caractérisé en ce qu'il comprend en outre:

d) un moyen de transfert de liquide (2) comprenant une aiguille d'introduction de liquide (7a) pouvant être reliée à un récipient dans lequel est stocké une solution de médicament, telle qu'une solution saline physiologique ou analogue, un troisième tube de liaison (8,9) relié à l'aiguille d'introduction (7a) de liquide et un cylindre de goutte à goutte (11) relié au troisième tube de liaison (8,9) vers le milieu du troisième tube de liaison;

e) dans ledit moyen de transfert de sang (3a, 3b) N aiguilles individuelles (17a) d'introduction de sang (N ≥ 2 et N > n) pouvant être reliées aux poches de sang (35a, 35b) dans lequel le sang est stocké à partir du sang du donneur pour être réinjecté au donneur, des moyens de goutte à goutte (12a, 12b) reliés aux aiguilles d'injection de sang (17a) et munis de moyens de filtration (14a, 15a, 16a) à l'intérieur de ceux-ci, et un quatrième tube de liaison (13a, 13b) relié aux moyens formant cylindres de goutte à goutte (12a, 12b); et

f) un moyen de stockage (6) d'une solution de lavage comprenant une poche de liqueur usée pour récupérer le liqueur usée après amorçage du dispositif de prélèvement de sang, et un cinquième tube de liaison (27, 28) pour l'acheminement du liqueur usée à l'intérieur de la poche de liqueur usée (26); ces éléments de stockage de sang individuels (5a,,5b) comprenant de préférence en plus une seconde poche (36a, 36b) reliée à la poche de prélèvement de sang (35a, 35b).

2. Dispositif de prélèvement de sang selon la revendication 1, comprenant un élément de connexion (41) relié aux troisième et quatrième tubes de liaison (8, 9 ; 13a, 13b), des moyens de connexion pour la mise en communication de l'élément de connexion (42, 43) et des premier, deuxième et cinquième tubes de liaison (21, 22 ; 37a, 37b ; 27, 28) les uns avec les autres, et un sixième tube de liaison (40) pour relier l'élément de connexion (41) aux moyens de connexion.

3. Dispositif de prélèvement de sang selon la revendication 2, dans lequel chacun des premier, deuxième, cinquième et sixième tubes de liaison est muni de moyens de pincement (A-H).

4. Dispositif de prélèvement de sang selon la revendication 1, 2, ou 3 dans lequel le moyen de stockage de la solution de lavage (6) comporte un élément (31) pour mettre en communication la poche de liqueur usée (26) avec le cinquième tube de liaison (27, 28).

5. Dispositif de prélèvement de sang selon l'une des revendications 1 à 4, dans lequel la poche de liqueur usée (26) comporte à l'intérieur un clapet anti-retour (33).

6. Dispositif de prélèvement de sang selon l'une des revendications 2 à 5, comportant en outre, n éléments de connexion (51a, 51b, 51c) pour une liaison amovible des éléments de stockage de sang individuels (5a, 5b) avec le moyen de connexion.

7. Dispositif de prélèvement de sang selon l'une des revendications 1 à 6, dans lequel le moyen de transfusion du sang est muni d'un instillateur cylindrique (61) qui est relié aux N aiguilles individuelles (N ≥ 2) d'injection de sang (62a, 62b) dans l'embranchement, la relation entre le nombre N des aiguilles d'injection de sang (62a, 62b) et le nombre n d'éléments de stockage de sang étant n ≥ N.

FIG_1

FIG_2

FIG_3

FIG_4

# FIG_5(a)

# FIG_5(b)

FIG_6(a)

FIG_7(a)

FIG_6(b)

FIG_7(b)

FIG_8

FIG_9

FIG_10

EP 0 314 016 B1

FIG_13

FIG_11

# FIG_12

FIG_14

FIG_15

(a)

(b)

(c)

(d)

EP 0 314 016 B1

25

FIG.16

FIG.17

(d)

400b    400c

400a

A    B    C

(c)

②    ④    ⑥

(b)

(a)

①    ③    ⑤    ⑦

A    B    C    D    D    D    D

400a    400b    400c    402a    402b    402c    402d

FIG.18